Europäisches Patentamt

European Patent Office

Office européen des brevets

(11) Publication number: **0 372 847**
**A2**

# (12) EUROPEAN PATENT APPLICATION

(21) Application number: 89312549.2

(22) Date of filing: 01.12.89

(51) Int. Cl.5: **C07C 67/00**

(30) Priority: 07.12.88 GB 8828616

(43) Date of publication of application:
**13.06.90 Bulletin 90/24**

(84) Designated Contracting States:
**AT BE DE ES FR GB IT LU NL SE**

(71) Applicant: **BP Chemicals Limited**
**Belgrave House 76 Buckingham Palace Road**
**London, SW1W 0SU(GB)**

(72) Inventor: **Pimblett, Gillian**
**5 Landseer Avenue West Ardsley Wakefield**
**West Yorkshire, WF3 1UE(GB)**
Inventor: **McLachlan, Kenneth Alan**
**The British Petroleum Company p.l.c.**
**Chertsey Road**
**Sunbury-on-Thames Middlesex TW16 LN(GB)**
Inventor: **Price, Peter John**
**The British Petroleum Company p.l.c.**
**Chertsey Road**
**Sunbury-on-Thames Middlesex TW16 LN(GB)**

(74) Representative: **Crack, Richard David et al**
**BP INTERNATIONAL LIMITED Patents**
**Division Chertsey Road**
**Sunbury-on-Thames Middlesex TW16**
**7LN(GB)**

(54) Ester production by hydrogenation of carboxylic acids and anhydrides.

(57) A carboxylic acid ester is produced at a selectivity of greater than 50% whilst producing the corresponding alcohol at a selectivity less than 10% from a carboxylic acid or anhydride thereof by reacting the acid or anhydride with hydrogen at elevated temperature in the presence as catalyst of a composition comprising a component (i) comprising at least one of Group VIII noble metal and/or a component (ii) comprising at least one of molybdenum, tungsten and rhenium and a component (iii) comprising an oxide of a Group IVb element. The process is particularly applicable to the hydrogenation of acetic acid to ethyl acetate.

EP 0 372 847 A2

# ESTER PRODUCTION BY HYDROGENATION OF CARBOXYLIC ACIDS AND ANYDRIDES

The present invention relates to the hydrogenation of carboxylic acids and their anhydrides to produce corresponding esters.

The hydrogenation of carboxylic acids to produce alcohols has long been known. Representative of the prior art may be mentioned US Patents Nos 4,446,073; 4,398,039; 4,104,478; 3,985,814 and 4,524,255; DE-A-2,605,107, GB-A-1534232 and GB-A-1551741.

US Patent No. 4,446,073 discloses the reduction of unsaturated carboxylic acids to esters or alcohols using a catalyst comprising low valence oxidation state cadmium and ruthenium and optionally a platinum group metal composited on a solid support.

US Patent No. 4,393,039 discloses the vapour phase hydrogenation of carboxylic acids to the corresponding alcohols in the presence of steam and a catalyst comprising the mixed oxides of ruthenium and at least one of cobalt, nickel, and optionally one of cadmium, zinc, copper, iron, rhodium, palladium, osmium, iridium and platinum. The catalyst may be supported on an inert carrier such as aluminates, silicates, titanates, zirconia and mixtures thereof. In the absence of steam carboxylic acid esters were also prepared.

US Patent No. 4,104,478 discloses the conversion of fatty acids into the corresponding fatty alcohols by hydrogenation over catalysts containing rhenium and ruthenium, rhodium, palladium or platinum.

US Patent No. 3,985,814 discloses a process for converting $-CO_2H$ groups to $-CH_2OH$ groups by hydrogenation using a platinum catalyst.

US Patent No. 4,524,225 discloses the hydrogenation of fatty acids using as catalyst combinations of copper, chromium, ruthenium, platinum, palladium and rhenium supported on alpha-alumina, theta-alumina, titanated alumina, titania or aluminium phosphate.

DE-A-2,605,107 discloses the hydrogenation of carboxylic acids to alcohols catalysed by palladium/rhenium combinations.

GB-A-1534232 discloses a process for the production of an alcohol having five or more carbon atoms by the catalytic hydrogenation of the corresponding carboxylic acid or of a lactone or anhydride thereof in the presence of water and/or other solvents and a catalyst comprising palladium and rhenium and an acid-resistant carrier, eg silica, alumina or activated charcoal.

GB-A-1551741 discloses a process for preparing butanediol(1,4) by hydrogenating maleic anhydride, maleic acid or a mixture thereof in the presence of a catalyst comprising an element of Group VIIA of the Mendeleef Periodic Table or a compound thereof and ruthenium, rhodium, palladium, osmium, iridium or platinum or a compound thereof or a mixture of such elements and compounds.

More recent patent publications include our EP-A-0198681 relating to the production of either ethanol from acetic acid or propanol from propionic acid in the vapour phase by contact with hydrogen and a catalyst comprising (i) a noble metal of Group VIII of the Periodic Table and (ii) rhenium, optionally supported on a high surface area graphitised carbon, a graphite, a silica, an alumina or a silica/alumina; EP-A-0198682 relating to the production of either an alcohol and/or a carboxylic acid ester from a $C_2$ to $C_{12}$ carboxylic acid by contact with hydrogen and a heterogeneous catalyst comprising (i) molybdenum or tungsten and (ii) a noble metal of Group VIII of the Periodic Table, optionally supported on a high surface area graphitised carbon, a graphite, a silica, and alumina or a silica/alumina; EP-A-0 210 795 relating to the production of an alcohol by hydrogenating a carboxylic acid in the presence of a reductively activated solid copper-containing catalyst additionally incorporating at least one of magnesium, a lanthanide metal or an actinide metal and EP-A-0 285 420 relating to the production from either a carboxylic acid or an anhydride thereof of the corresponding alcohol and/or carboxylic acid ester by reaction with hydrogen in the presence as catalyst of a composition comprising an alloy of (i) at least one noble metal of Group VIII of the Periodic Table and (ii) at least one metal capable of alloying with the noble metal, for example silver, optionally supported on a high surface area graphitised carbon, a graphite, an activated carbon, a silica, an alumina or a silica/alumina.

We have now unexpectedly found that by a judicious choice of catalyst and reaction conditions $C_2$ to $C_{12}$ carboxylic acids and their anhydrides can be hydrogenated preferentially to their corresponding carboxylic acid esters, rather than the corresponding alcohols.

Accordingly, the present invention provides a process for the production from a $C_2$ to $C_{12}$ carboxylic acid or an anhydride thereof of the corresponding carboxylic acid ester at a selectivity of greater than 50% whilst producing the corresponding alcohol at a selectivity less than 10% which process comprises reacting at elevated temperature the acid or anhydride with hydrogen in the presence as catalyst of a composition comprising a component (i) comprising at least one noble metal of Group VIII of the Periodic Table of the

2

Elements and/or a component (ii) comprising at least one of molybdenum, tungsten and rhenium, and a component (iii) comprising an oxide of an element of Group IVb of the Periodic Table of the Elements, under reaction conditions such that the conversion of the acid or anhydride to hydrogenation products is maintained below a value consistent with achieving the aforesaid selectivities.

The Periodic Table of the Elements referred to in this specification is that to be found in the Handbook of Chemistry and Physics, 63rd edition.

It is believed, though the belief is not to be interpreted as binding in any manner, that the Group IVb-containing catalyst of the present invention behaves quite differently to the carbon and silica supported catalysts of, for example EP-A-0 198 681, in that the latter catalysts produce an alcohol as the first-formed product and the ester as the second-formed product arises from the reaction of the alcohol with unreacted carboxylic acid or anhydride. The ratio of ester to alcohol is limited in this case by the thermodynamic equilibrium of the esterification reaction, typically in the case of acetic acid hydrogenation to an ethyl acetate to ethanol ratio of about 1.9:1. In contrast, the catalyst of the present invention is believed to form the ester as the first-formed product and thereafter the ester is hydrogenated to the alcohol. Whether this theory be true or not, it is nevertheless observed that by restricting the acid conversion the formation of alcohol can be kept to very low levels or eliminated completely. Thus, it is possible to produce from acetic acid, for example, ethyl acetate at selectivities greater than 90% with accompanying ethanol selectivities of 5% or less and in some cases zero. The capability of the process of the invention to produce an ester free from alcohol provides the advantage that the generally difficult separation of the alcohol from the ester can be avoided. Moreover, the selective production of esters from carboxylic acids is a desirable objective in its own right because it avoids the requirement for an alcohol, which is reacted with an acid in conventional esterification processes for producing esters.

Apart from the catalyst, the principal parameter influencing the selectivity to ester formation is the conversion. The conversion below which the desired selectivities to ester are achieved will depend on the catalyst used and on the nature of the carboxylic acid to be hydrogenated. Typically, for acetic acid hydrogenation, for example, it may be necessary to limit the acid conversion to less than 90%. By limiting acid conversions to achieve high selectivities to ester it is possible to recycle the unconverted acid and to reduce separation problems.

The process of the invention is applicable to both $C_2$ to $C_{12}$ carboxylic acids and their anhydrides. The carboxylic acid or anhydride thereof may be either saturated or unsaturated. Mono-, di- or polybasic carboxylic acids and their anhydride derivatives may be employed. Suitable monobasic acids include acids having the formula R-COOH, wherein R is a substituted or unsubstituted aliphatic or aromatic group, which acids are hydrogenated to esters of the formula RCOOR. Suitably the group R may be a $C_1$ to $C_{12}$, typically a $C_1$ to $C_5$ alkyl group. Examples of suitable monobasic acids include acetic acid, propionic acid, butyric acids and heptanoic acids. Preferred monobasic acids include acetic acid from which there is obtained ethyl acetate and propionic acid from which there is obtained propyl propionate.

Alternatively, dibasic acids, polybasic acids and their anhydrides may be employed. Suitable dibasic acids may be both saturated or unsaturated. Examples of suitable dibasic acids include glutaric acid, glutaric anhydride, adipic acid, adipic anhydride, succinic acid, succinic anhydride, maleic acid and maleic anhydride. Preferred are $C_4$ dibasic acids and their anhydrides. Mixtures of acids, anhydrides and acids/anhydrides may also be employed.

Hydrogen is commercially available on a large scale and may be used with or without further purification depending upon any impurities present. A desirable purification may be removal of carbon monoxide.

As catalyst there is used a composition comprising a component (i) comprising at least one noble metal of Group VIII of the Periodic Table and/or a component (ii) comprising at least one of molybdenum, tungsten and rhenium and a component (iii) comprising an oxide of an element of Group IVb of the Periodic Table of the Elements. The noble metals of Group VIII of the Periodic Table for the purpose of this specification at least are palladium, platinum, rhodium, ruthenium, osmium and iridium. Of the aforesaid noble metals at least one of palladium, rhodium and ruthenium is preferred, and at least one of palladium and ruthenium is more preferred.

Component (ii) may be present as a metal, a metal oxide or mixtures thereof. Preferably component (ii) comprises rhenium.

For the purpose of this specification at least the elements of Group IVb of the Periodic Table of the Elements are titanium, zirconium and hafnium. The elements of Group IVb are present in the catalyst in the form of their oxides. A preferred oxide is titania. Titania can exist in the forms of anatase titania and rutile titania. There are indications that anatase titania or a mixture of anatase and rutile titania principally comprising anatase titania may be preferred.

3

The catalyst may be used in the form of component (i) and/or component (ii) supported on component (iii) or in the form of component (i) and/or component (ii) and component (iii) supported on an inert support. Suitable inert supports include high surface area graphitised carbons (HSAGs), graphites, activated carbons, silicas, aluminas and silica/aluminas.

The catalyst may incorporate one or more further components. Thus, one or more metals selected from Groups IA or Group IIA of the Periodic Table of the Elements may be incorporated. A suitable metal is potassium.

Suitably the catalyst comprises from 0.1 to 20%, preferably from 1 to 10%, by weight of component (i) and/or from 0.1 to 20%, preferably from 1 to 10%, by weight of component (ii).

Examples of suitable catalysts include palladium/titania, rhenium/titania, palladium/rhenium/titania and ruthenium/titania.

The catalysts for use in the process of the invention may suitably be prepared by impregnation. One method of preparing the catalyst comprises impregnating component (iii) with an aqueous or non-aqueous solution of soluble thermally decomposable compounds of component (i) and/or component (ii) and thereafter thermally decomposing the thermally decomposable compounds to the metal and/or metal oxide. For further details of such processes the reader is referred to our EP-A-0 198 681 and to the examples accompanying the present specification.

Before use in the process of the invention the catalyst is preferably activated by contact at elevated temperature with hydrogen, optionally also containing an inert gas, for example nitrogen, for a suitable period, for example from 1 to 24 hours. Activation may be accomplished by the catalyst manufacturer or the process operator immediately prior to operation of the process or both.

As regards the process for producing the ester, this may be operated either in the liquid phase or the vapour phase. It may be operated either batchwise or continuously. Continuous operation is preferred, with recycle of unconverted carboxylic acid or anhydride being a desirable option. The catalyst may be employed in the form of a fixed bed, a moving bed or a fluidised bed.

The elevated temperature may suitably be in the range from 50 to 300°C, though this will depend to some extent on the nature of the carboxylic acid reactant, the catalyst employed and the mode of operation. For monobasic acids the elevated temperature will generally be in the range from 100 to 300°C, preferably from 150 to 250°C. Using unsaturated dibasic acids the elevated temperature will generally be in the range from 50 to 350°C, preferably from 150 to 300°C. The pressure will generally be in the range from 1 to 300 barg, though again this will depend on the aforementioned factors. For monobasic acids it will suitably be less than 50 barg and for unsaturated dibasic acids it will generally be in the range from 10 to 150 barg.

The invention will now be further illustrated by reference to the following Examples.

In the Examples a number of terms requiring further definition are employed. These are as follow:-

Nominal loading is defined as weight of metal (not metal compound) added to the support expressed as a percentage of the weight of the support.

WHSV is the Weight Hourly Space Velocity, which is defined as the number of kg of liquid feed per kg catalyst per hour.

LHSV is the Liquid Hourly Space Velocity, which is defined as the number of litres of liquid feed per litre of catalyst per hour.

Conversion is defined as the proportion of carboxylic acid converted to products.

Selectivity is defined as the proportion of reacted acid which is converted to a particular product.

(I) Catalyst Preparation

Catalyst A

An aqueous solution containing dissolved ruthenium nitrosyl nitrate was added to high surface area titanium oxide and the mixture was left to stand overnight. The water was removed on a rotary evaporator and the impregnated titania was dried overnight at 110°C in an oven. The amounts of various components were chosen to give a nominal loading of 2% Ru.

Catalyst B

The preparative procedure used for Catalyst A was followed. The resulting composition was then transferred to a glass tube and heated in a stream of hydrogen using the following conditions: from room temperature to 500°C over a period of five hours then two hours at 500°C followed by cooling to room temperature. The gas flow was then changed to nitrogen for a few hours.

Catalyst C

The procedure used for Catalyst B was followed except that the nominal ruthenium loading chosen was 1%. X-ray diffraction analysis of this catalyst showed it to comprise predominantly anatase titania with no evidence of rutile titania. The surface area measured by the BET method was $108m^2/g$. The ruthenium content measured by x-ray fluorescence (XRF) was 1.5% Ru.

Catalyst D

An aqueous solution of palladium nitrate was added to a sample of anatase titanium oxide powder (supplied by Tioxide) whose surface area was $200m^2/g$ as measured by the BET method. The water was removed on a rotary evaporator and the composition was dried at 100°C in a vacuum oven overnight. The composition was pelleted and the pellets were crushed and sieved to a particle size range of 0.5mm - 1mm. The amounts of various components were chosen to give a nominal palladium loading of 2.5% (2.0% measured by x-ray fluorescence (XRF)).

Catalyst E

The procedure used for Catalyst D was followed except that instead of anatase, rutile titanium oxide (supplied by Tioxide) was used with a BET surface area of $50m^2/g$ (Pd 2.1% by XRF).

Catalyst F

The procedure used for Catalyst D was followed except that rhenium oxide $Re_2O_7$ was used instead of palladium nitrate. The amounts of the various components were chosen to give a nominal rhenium loading of 5% (8% measured by XRF).

Catalyst G

The procedure used for Catalyst E was followed except that rhenium oxide $Re_2O_7$ was used instead of palladium nitrate. The amounts of the various components were chosen to give a nominal rhenium loading of 5% (8% measured by XRF).

Catalyst H

The procedure used for Catalyst D was followed and the resulting composition was heated in a flow of nitrogen using the following conditions: the temperature was raised to 300°C over 6 hours, held at 300°C for 10 hrs then cooling to room temperature took place. The composition was added to aqueous rhenium oxide and the water was removed using a rotary evaporator. The catalyst was dried, pelleted, crushed and sieved as described for Catalyst D. The amounts of the various components were chosen to give a nominal loading of 2.5% Pd, 5% Re (2.4% Pd, 3.5% Re by XRF).

Catalyst I

A solution of palladium acetate in ethyl acetate was added to a sample of anatase titania (0.5 - 1.0mm pellets) (supplied by Degussa) whose surface area was $57m^2/g$ as measured by the BET method. The ethyl acetate was removed on a rotary evaporator and the composition was dried at 100°C in a vacuum oven overnight. The resulting composition was heated in a flow of nitrogen using the following conditions: the temperature was raised to 300°C over 6hrs, held at 300°C for 10hrs and then cooled to room temperature.

The resulting composition was added to a solution of rhenium oxide in ethyl acetate and the ethyl acetate was removed using a rotary evaporator. The catalyst was dried in a vacuum oven overnight. The amounts of the various components were chosen to give a nominal loading of 2.5% Pd, 5% Re.

Catalyst J

The procedure for catalyst I was followed except anatase titania (0.5 - 1.00 mm pellets) (supplied by Norton) whose BET surface area was 178 m²/g was used.

(II) Catalyst Testing

Method

2 - 2.5mls of catalyst was loaded into a corrosion resistant stainless steel tube of internal diameter 6 - 7mm, and the reactor tube assembly placed in a horizontal tubular furnace. The catalyst was then activated by heating at atmospheric pressure in a flow of hydrogen. After activation, the catalyst was cooled in hydrogen to the desired reaction temperature. A mixture of acetic acid vapour and hydrogen was then passed over the catalyst, and the pressure was adjusted to the required value using a back-pressure regulator. The vapour/hydrogen mixture was formed in a vapourising zone, to which acetic acid liquid and hydrogen gas were separately metered. The product vapours and gases leaving the reactor were sampled and analysed by gas-liquid chromatography (GLC). The temperature was measured by means of a thermocouple inserted into the catalyst bed.

The main product is ethyl acetate with some ethanol at high conversions and some ethane and methane byproducts.

Examples 1 to 3

Catalyst activation was carried out by heating in hydrogen to 280°C over 2 hours and then holding at 280°C for 2 hours.

Acetic acid was hydrogenated over Catalyst A(Ex.1), B(Ex.2) and C(Ex.3) at 230°C, 10 barg, an LHSV of 1 and a hydrogen to acetic acid mole ratio of 10:1.

The results are reported in Table 1.

Table 1

| Example | Catalyst | Conversion (%) | Selectivities (%) | | | |
|---------|----------|----------------|------------------|---------|--------|---------|
| | | | ethyl acetate | ethanol | ethane | methane |
| 1 | A | 61.6 | 62.3 | 0 | 37.7 | 0 |
| 2 | B | 42.0 | 50.2 | 0 | 49.8 | 0 |
| 3 | C | 32.7 | 97.9 | 2.1 | 0 | 0 |

Examples 4 to 8

Catalyst activation was carried out by heating in hydrogen to 300°C over two hours and then holding at 300°C for 2 hours.

Acetic acid was hydrogenated over the Catalysts D (Ex.4), E(Ex.5), F(Ex.6), G(Ex.7) and H(Ex.8) at a hydrogen:acetic acid mole ratio of 10:1, a pressure of 10 barg and at the temperatures shown in Table 2.

The results are reported in Table 2.

Table 2

| Example | Catalyst | Temperature (°C) | LHSV | Conversion (%) | Selectivities (%) | | |
|---|---|---|---|---|---|---|---|
| | | | | | ethyl acetate | ethanol | others* |
| 4 | D | 202 | 0.68 | 29 | 94 | 0 | 5 |
| 5 | E | 202 | 0.68 | 7 | 89 | 0 | 10 |
| 6 | F | 200 | 0.68 | 40 | 96 | 1 | 4 |
| 7 | G | 202 | 0.68 | 13 | 91 | 0 | 7 |
| 8 | H | 208 | 1.34 | 65 | 92 | 5 | 3 |
| CTA | | 232 | 0.68 | 0.9 | 0 | 0 | 100 |

*Others = methane, ethane, acetaldehyde and acetone.

Example 9

Catalyst activation was carried out by heating in hydrogen to 300°C over two hours and then holding at 300°C for two hours.

Acetic acid was hydrogenated over the catalyst J (Ex. 9) at a hydrogen:acetic acid mole ratio of 10:1, a pressure of 10 barg and at the temperature shown in Table 3.

The results are reported in Table 3.

Table 3

| Example | Catalyst | Temperature (°C) | LHSV | Conversion (%) | Selectivities (%) | | |
|---|---|---|---|---|---|---|---|
| | | | | | ethyl acetate | ethanol | others* |
| 9 | J | 230 | 1.34 | 28 | 86 | 0 | 14 |

* Others = methane, ethane, acetaldehyde and acetone.

Examples 10 - 11

Catalyst activation was carried out by heating in hydrogen to 300°C over two hours and then holding at 300°C for two hours.

Acetic acid was hydrogenated over the catalyst H at an acetic acid LHSV = 1.34, a pressure of 10 barg, at a temperature of 228°C and hydrogen:acetic acid mole ratios shown in Table 5.

The results are reported in Table 5. These results show increased selectivity to ethyl acetate at lower conversions of acid.

Table 5

| Example | Catalyst | H₂:AcOH mole ratio | Conversion (%) | Selectivities (%) | | |
|---|---|---|---|---|---|---|
| | | | | ethyl acetate | ethanol | others* |
| 10 | H | 7:1 | 80 | 86 | 9 | 6 |
| 11 | H | 6:1 | 35 | 95 | 2 | 3 |

\* Others = methane, ethane, acetaldehyde and acetone.

Example 12

Catalyst activation was carried out by heating in hydrogen to 300°C over two hours and then holding at 300°C for two hours.

Acetic acid was hydrogenated over the catalyst I at an acetic acid LHSV = 1.34, a pressure of 10 barg, at the temperatures and hydrogen:acetic acid mole ratios shown in Table 6.

The results are reported in Table 6.

Table 6

| Ex | Catalyst | H₂:AcOH mole ratio | Temperature (°C) | Conversion (%) | Selectivities (%) | | |
|---|---|---|---|---|---|---|---|
| | | | | | ethyl acetate | ethanol | others* |
| 12 | I | 2.5:1 | 231 | 39 | 85 | 6 | 9 |

\* Others = methane, ethane, acetaldehyde and acetone.

Comparative Test A

The anatase titanium oxide as used in Catalysts D, F and H was tested for acetic acid hydrogenation after the activation procedure of Examples 4 to 8.

The results are reported in Table 2. They show that titanium oxide alone has almost negligible activity for acetic acid hydrogenation to ethyl acetate.

This is not an example according to the present invention and is included only for comparative purposes.

**Claims**

1. A process for the production from a $C_2$ to $C_{12}$ carboxylic acid or an anhydride thereof of the corresponding carboxylic acid ester at a selectivity of greater than 50% whilst producing the corresponding alcohol at a selectivity less than 10% which process comprises reacting at elevated temperature the acid or anhydride with hydrogen in the presence as catalyst of the composition comprising a component (i) comprising at least one noble metal of Group VIII of the Periodic Table of the Elements and/or a component (ii) comprising at least one of molybdenum, tungsten and rhenium and a component (iii) comprising an oxide of an element of Group IVb of the Periodic Table of the Elements, under reaction conditions such that the conversion of the acid or anhydride to hydrogenation products is maintained below a value consistent

8

with achieving the aforesaid selectivities.

2. A process according to claim 1 in which component (iii) comprises titania.

3. A process according to claim 2 in which the titania principally comprises anatase titania.

4. A process according to any one of the preceding claims in which the catalyst comprises from 0.1% to 20% of component (i).

5. A process according to any one of the preceding claims in which component (i) comprises at least one of palladium, rhodium and ruthenium.

6. A process according to claim 5 in which component (i) comprises at least one of palladium and ruthenium.

7. A process according to any one of the preceding claims in which the catalyst comprises from 0.1% to 20% of component (ii).

8. A process according to any one of the preceding claims in which component (ii) comprises rhenium.

9. A process according to any one of the preceding claims in which the catalyst further comprises at least one metal selected from Group IA or Group IIA of the Periodic Table of Elements.

10. A process according to any one of the preceding claims in which the catalyst is prepared by impregnation from a non-aqueous solution.

11. A process according to any one of the preceding claims in which the carboxylic acid comprises acetic acid, propionic acid, butyric acids or heptanoic acids.

12. A process according to any one of the preceding claims in which the catalyst is activated before use by contacting with hydrogen at elevated temperature.

13. A catalyst for use in the process of claim 1 comprising a component (i) comprising at least one noble metal of Group VIII of the Periodic Table of the Elements and/or a component (ii) comprising at least one of molybdenum, tungsten and rhenium, and a component (iii) comprising an oxide of an element of Group IVb of the Periodic Table of the Elements.